# EUROPEAN PATENT SPECIFICATION

(11) **EP 3 490 528 B1**
(45) Date of publication and mention of the grant of the patent: **17.02.2021**
(21) Application number: 17757886.1
(22) Date of filing: 27.07.2017
(51) Int. Cl.: A61Q 1/14, A61K 9/00, A61K 47/44, A61Q 19/00, A61K 8/14, A61K 8/22, A61K 8/36, A61K 8/37, A61K 8/55, A61K 8/02, A61K 8/92, A01N 25/04, A01N 59/00, A61K 9/107, A61K 31/357, A61K 33/40, A61K 9/127

(54) **OIL AND DEVICE FOR CLEANING THE OCULAR AND PERIOCULAR AREA**
ÖL UND VORRICHTUNG ZUR REINIGUNG DES OKULAREN UND PERIOKULAREN BEREICHS
HUILE ET DISPOSITIF POUR LE NETTOYAGE DE LA ZONE OCULAIRE ET PÉRI-OCULAIRE

(30) Priority: 27.07.2016 IT 201600078872
(43) Date of publication of application: 05.06.2019
(73) Proprietor: FB VISION S.p.A., 63074 San Benedetto del Tronto (IT)
(72) Inventor: BELLINI, Francesco, Calgary, Alberta T2P OC3 (CA); MARCHETTI, Marco, 63074 San Benedetto del Tronto (IT); CARBONI, Paolo, 63074 San Benedetto del Tronto (IT); MAGAGNINI, Mattia, 60127 Castelfidardo (IT)
(74) Representative: Coppo, Alessandro
(86) International application number: PCT/IB2017/054567
(87) International publication number: WO 2018/020456

(56) References cited:
- EP-A1- 2 149 598
- WO-A1-96/25409
- WO-A1-2006/087359
- WO-A1-2008/056389
- WO-A1-2014/186399
- WO-A2-2007/046122
- DE-A1-102008 032 327
- ES-A1- 2 577 885
- GB-A- 2 431 581
- US-A1- 2006 074 129
- US-A1- 2011 150 959
- US-A1- 2015 320 594

## Description

### FIELD OF THE INVENTION

The present invention relates to an ophthalmic formulation based on an ozonized oil and a device for cleaning the ocular and periocular area.

The present invention originates in the cosmetic and ophthalmic fields and in particular in the field of medical-surgical devices for care and hygiene of the eyes and periocular areas.

Specifically, the present invention relates to a formulation based on a selected ozonized agent and a device soaked with ozonized oil intended to lubricate, hydrate and/or disinfect the eyes and/or cleanse the periocular area.

### STATE OF THE ART

Ocular hygiene helps to protect eye health and to prevent pathologies of the eye anterior segment.

Every day eyes and their skin adnexa are exposed to the action of agents exerting an irritant and inflammatory action.

The agents causing this irritating action fall into three main categories: (i) environmental agents, (ii) infectious agents, (iii) external agents.

Environmental agents include chemical or physical pollutants present in the atmosphere; Infectious agents include bacteria, viruses and fungi present in the environment; external agents include substances or products that are applied to the eye or to the periocular area for aesthetic-cosmetic purposes, such as contact lenses, eyeshadows and blushes.

Single or combined action of these external agents on the anatomical structures of the eye is the main cause of irritating phenomena and of the most common eye diseases.

Effective prevention of these diseases can be achieved by adopting correct eye hygiene measures, which are designed to remove irritating substances and infectious agents from the eyes and to clean the periocular areas.

The easiest cleansing action on the eyes is achieved by washing. This simple common practice removes some of the agents responsible for irritation of the eyes and periocular areas as it eliminates some of the causes that promotes the onset of local inflammatory or infectious phenomena.

However, in many cases washing is not enough to effectively remove eye secretions and external agents deposited on eyelashes and eyelids.

Therefore, for deeper cleansing of the eye area, specific detergent products can be used, such as cleansing solutions or gels, disposable wipes soaked with emollient substances.

Daily use of these eyewash products is also advisable for children because these younger subjects have an immature immune system that often is still unable to respond adequately to the action of infectious agents.

It is also important to note that children and infants are more exposed to eye infections because they touch and rub their eyes without worrying about adopting the most common personal hygiene rules. Under these conditions, availability of specific products for ocular hygiene becomes more important.

In fact, an incorrect or inadequate eye hygiene is still currently one of the major causes of infections, irritations and inflammation of the eyes, especially in the case of younger subjects.

At present, eye hygiene and deep cleansing of periocular areas are performed by applying eyedrops, palpebral wash solutions that contain detergent and emollient substances, or using medicated wipes or gauze. The latter find specific application in eye hygiene of children and feminine subjects, who use eye shadows, mascara and eyeliners.

Gauze and detergent handkerchiefs are used for eyelids and eyelashes cleansing by applying a slight surface rubbing action while keeping the eye closed. This eliminates external agents, such as eye makeup and pollutants from the treated surfaces, and at the same time causes expulsion of the eyelid glands contents.

However, available eye care remedies do not perform a fully satisfactory disinfectant-emollient action because the addition of disinfectants in bactericidal concentrations to eye cleansers reduces the emollient effect and can cause redness or allergies. On the other hand, disinfectant agent-free detergent products only perform a cleansing and soothing action, and are inadequate in preventing the most common palpebral and conjunctival diseases such as blepharitis, conjunctivitis, blepharo-conjunctivitis, and tear-sac infection.

Some ophthalmic products with ozonized action, despite providing a suitable disinfectant, antimicrobial action, have the disadvantage of irritating the eye and sensitizing delicate periocular areas.

The appearance of redness and irritation of the eye, following application of substances potentially suitable for disinfecting and cleansing the eye and its adnexa, prevent their ophthalmic use.

In addition, while some ophthalmic products have been found to provide a suitable ozonized action, they have the disadvantage of limited stability and their antimicrobial action is reduced in a short period of time. Ozonized pharmaceutical and cosmetic formulations have been disclosed in US 2006/074129 A1, WO 2007/046122 A2, WO 2008/056389 A1, WO 2006/087359 A1, EP 2 149 598 A1,GB 2 431 581 A, WO 2014/186399 A1 and US 2011/150959 A1.

Currently, the need is felt for new ophthalmic formulations with an effective detergent, hygienic and/or antimicrobial action, whose application in the eye and periocular area does not lead to local side effects, such as irritation and sensitization of the treated tissues.

One of the objects of the present invention is therefore to provide a formulation for the hygiene of the eye and the periocular area with detergent and antimicrobial action, whose local use is substantially free of local side effects or significantly reduces the onset thereof.

Another object of the invention is to provide a device for eye care that is easy to use and prevents or treats the more common diseases of the eye without using antibiotics whose ophthalmic use causes irritation or phenomena of sensitization of the eye and periocular area.

### SUMMARY

While performing ophthalmic research activities, the inventors have identified a composition based on selected ozonized oils, whose formulation avoids or reduces substantially the local side effects when applied to the eye or periocular area.

In particular, the present invention originates from the finding that topical ophthalmologic use of ozonized oils is accompanied by appearance of irritating and sensitizing phenomena of the epidermis, that preclude or substantially reduce ophthalmic use thereof.

Starting from this finding, the inventors have surprisingly found that selecting specific ozonized oils and formulating the same with phospholipids or nanomicelles not only reduces considerably skin irritability and sensitization phenomena but also increases the bactericidal and detergent activity of unformulated ozonized oil.

Therefore, according to a first aspect, the present invention provides a cosmetic use of a composition containing ozonized oil for cleansing and/or lubrication of the ocular and/or periocular area, wherein said ozonized oil is carried by liposomes or phospholipid micelles and comprises ozonized fatty acids and/or fatty acid triglycerides selected from the group consisting of mono-unsaturated fatty acids C₁₄-C₂₄ polyunsaturated fatty acids C₁₈-C₂₂ and mixtures thereof and/or their triglycerides with from 1 to 3 glycerol.

According to some embodiments, the composition contains excipients for topical application on the surface of the eyeball and/or on the eyelids, and/or eyelashes. According to some embodiments, the ozonized oil contained in the composition is formulated as eye drops, ophthalmic gel or ointment for cosmetic or medical uses.

According to another aspect, the invention provides a composition comprising ozonized oil for use in the disinfection and/or healing of an epithelial or keratin structure of the eye or the periocular area, wherein said ozonized oil is conveyed by liposomes or phospholipid micelles and comprises ozonized fatty acids and/or fatty acid triglycerides selected from the group consisting of mono-unsaturated fatty acids C₁₄-C₂₄ polyunsaturated fatty acids C₁₈-C₂₂ and mixtures thereof and/or their triglycerides with from 1 to 3 glycerol molecules.

The composition of the invention can be applied directly on the eye and/or the periocular area.

According to some embodiments, both for cosmetic and ophthalmic medical uses, the composition of the invention is in the form of a phospholipid or liposomal emulsion.

Ways of employment of the compositions of the invention in cosmetic and/or medical field are defined in claims 2-6 and 8-12, respectively.

According to some embodiments, a device or kit is provided comprising a substrate wetted with a composition containing ozonized oil formulated with liposomes for the cosmetic and/or medical uses as previously described. According to preferred embodiments, the device of the invention is a medical-surgical device.

Embodiments of the device of the invention are defined in the appended claims 13, 14.

### DETAILED DESCRIPTION OF THE INVENTION

The applicant of the present invention has found that, by formulating selected ozonized oils with liposomes, the affinity of ozonized oils with the periocular area of the corneum layer increases and an unexpected reduction of local phenomena of sensitization and irritability of the treated epithelial tissue occurs, as well as an increase of the bactericidal and cleansing activity of the ozonized oils.

According to certain aspects, the invention provides a liposome composition for ophthalmic and/or cosmetic use which is highly tolerable and produces an effective disinfectant and/or cleansing action of the eye and periocular area. The applicant has found that phospholipid micelles or liposomes contained in the formulation, acting as vehicles highly compatible with keratinic skin structures, increase the affinity of selected ozonized oils with respect to the corneum layer of the periocular area and increase local tolerability and bactericidal and cleansing activity of ozonized oils.

According to certain aspects, the cosmetic use of a liposome or phospholipid composition based on ozonized oil is provided according to any one of claims 1-6 or, alternatively, the medical-ophthalmic use of a liposome or phospholipid composition based on ozonized oil according to any one of claims 7-12. Typically, the composition of the invention comprises an oxidizing oil based on an ozonized oil as active component comprising an ozonized fatty acid, wherein said fatty acid is mono-unsaturated C₁₄-C₂₄ or with 14 to 24 carbon atoms, or C₁₆ - C₂₂ or C₁₆ - C₁₈.

By way of example, suitable mono-unsaturated fatty acids include myristoic, palmitoleic, oleic, cetoleic, nervonic acid and mixtures thereof.

According to some embodiments, the ozonized oil contained in the composition of the invention comprises an ozonized fatty acid in which said fatty acid is poly-unsaturated C₁₈-C₂₂.

By way of example, suitable poly-unsaturated fatty acids include linoleic acid, arachidonic acid, EPA, DHA, DPA (fatty acids with double bonds in position Ω-3, Ω-6) and mixtures thereof.

According to some embodiments, the ozonized oil comprises a mixture of:
- mono-unsaturated fatty acids C₁₆-C₁₈, such as palmitoleic acid and oleic acid, for example, in a quantity of 35 to 50% by weight, relative to the total quantity of fatty acids,
- poly-unsaturated fatty acids C₁₆-C₁₈, for example, linoleic acid, gamma linolenic acid in a quantity ranging from 30 to 45% by weight, relative to the total amount of fatty acids.

Typically, the ozonized oil of the formulation of the composition of the invention is a reaction or adduct product of ozone with one or more unsaturated fatty acids.

In some embodiments, the ozonized oil origins from an ozonization process of a vegetable oil, such as palm oil, olive oil, sunflower oil, corn oil, various seeds oil, and mixtures thereof. By way of example, ozonized oils used in the invention are based on oleic and linoleic acids.

According to some embodiments, the native oils are triglycerides with one or more of the fatty acids previously mentioned.

According to some embodiments, the ozonized oil comprises or is contained in phospholipid micelles. The liposomal dispersion increases the effectiveness of the ozonized oil because the micelles match with the cell membranes of the microorganisms with which they come into contact after application on an ocular or periocular area, increasing the contact with ozonides.

Typically, the ozonized oil contained in the composition of the invention is carried or embedded within the lipid component of liposomes or phospholipid micelles.

Suitable liposomes include vesicles or microspheres based on mono- or multilayered phospholipids.

Typically, the phospholipids used as carriers within the scope of the invention comprise a fatty acids based hydrophobic tail and a hydrophilic head comprising a phosphate group bound to a polar group, such as choline, serine, ethanolamine. According to one embodiment, the phospholipids include phosphatidylcholine.

Suitable liposomes may contain other lipid components, such as cholesterol, stearylamine, soy lecithin.

Typically, phospholipid vesicles forming liposomes may include a double layer of closed phospholipids and/or cholesterol.

The applicant has found that liposomes, in particular those containing phosphatidylcholine based phospholipids, have high compatibility with cell membranes and low cytotoxicity.

When they come into contact with bacteria contaminating the periocular area, phospholipids or phospholipid micelles combine with the cell membranes of the bacteria, and release the particles of ozonized oil causing oxidation and lysis of the bacterial cells and reducing the bacterial count of the treated area. According to some embodiments, the liposomes comprise phospholipid vesicles of the type previously described and having a diameter of 5 to 2000 nm, 10 to 500nm, preferably 15 to 200nm, 20 to 100nm.

According to some embodiments, liposomes have spherical shape with a nucleus containing ozonized oils.

Suitable liposomes for the applications of the invention may have variable dimensions and shapes. For example, MLV liposomes with large multilamellar vesicles can be used, such as having size ≥ 0.5µm, SUV liposomes with small unilamellar vesicles, for example, having sizes of 20 to 100 nm, LUV liposomes with large multilamellar vesicles having dimensions ≥ 0.1 µm.

The liposomal compositions of the invention or liposomal emulsions with ozonized oil can be obtained with conventional preparation technologies, such as thin layer evaporation or centrifugation or ultracentrifugation technologies.

By way of example, a process for preparing an ozonized oil carried with phospholipids, comprises contacting an ozonized oil with phospholipids, typically based on phosphatidylcholine and a fatty acid, in a reactor provided with stirrer and cold mixing, for example, at a speed of 700 to 1000 rpm up to the incorporation of ozonized oil in phospholipid micelles.

According to some embodiments, the manufacturing process comprises adding phospholipids into a reactor in an organic solvent, such as methanol or chloroform or a mixture thereof, and then adding the ozonized oil, evaporating at reduced pressure, for example 40000psi, keeping the system rotating until a film is formed that can be further dried by using a mechanical pump.

The obtained lipid film is then rehydrated with a buffer solution. The mixture is then placed in a stirrer until it becomes homogeneous. Subsequently, the resulting emulsion is extruded through the passage under pressure, e.g. in a systems of partitions that reduces the liposome diameter and homogenizes the emulsion itself, thereby obtaining SLV liposomes. Thereafter, the resulting emulsion is sterilized for example by filtration.

According to some embodiments, liposomes with size ≤200 nm are obtained through extrusion and filtration phases, because the more phospholipids are small, the more the contact surface between the cell membrane of the microorganism and the phospholipid itself increases.

According to some embodiments, the phospholipids or micelles of the composition comprise or contain or incorporate ozonides within a concentration of 3% to 60% (w/w), 6 to 30%, 10 to 20%, compared to other components of the composition or phospholipid.

According to an aspect of the invention, an ozonized oil based composition is provided according to any one of the embodiments previously described, for medical use, specifically for ophthalmic use.

The ozonized oil of the composition for ophthalmic use can be applied as an antimicrobial and/or regenerating or healing agent of damaged keratinic structures. When the ozonized oil is contacted with exudates coming from affected tissues, it slowly release O₃ and generates lipoperoxides that exert a stimulating activity on the cells by accelerating the tissue repair and healing process. The ozonized oil accelerates the healing of the ocular and/or periocular zone affected tissues, by stimulating the expression of tissue growth factors, such as TGF-β, VEGF, PDGF.

Antimicrobial action in included among the applications in the medical field of the ozonized oil or the composition of the invention. The ozonized oil based composition applied to the ocular or periocular area reduces the total viable counts by releasing O₃ and exerting an oxidation action on the bacteria present on the skin and/or the keratinic structures of the periocular area, and also by exerting an inactivation action of enzymatic pathways on the epithelial surfaces of the ocular surface.

Furthermore, the ozonized oil of the invention applied to the epithelium of the ocular and periocular area finds application as an immunostimulant. This activity is mediated by lipoperoxides, which are generated by ozonides and act as a stimulant on fibroblasts and keratinocytes.

According to a further aspect, the present invention provides a method for disinfecting or healing treatment of the eye and periocular area of an individual, said method comprising application on the eye or periocular area of a therapeutically effective amount of a composition according to any one of the embodiments herein described.

In certain embodiments, application in the eye comprises the instillation of one or more drops, e.g. 2-5 drops, in an eye of an ophthalmic composition according to any one of the embodiments described herein.

The ozonized oils used within the scope of the invention can be obtained by conventional procedures and techniques.

Ozone is an allotropic form of oxygen (O₃) that can be added to the double bonds of an organic molecule with the formation of cyclic groups, called ozonides, in order to make it more stable.

Typically, the fatty acid ozonization reaction occurs in two phases, in the first one ozone is added to the double bond with the formation of a compound called molozonide, while in the second one the molozonide is rearranged into the ozonide/trioxolane.

Ozonized oils suitable for the applications according to the present invention can can be obtained starting from fatty acids of the type described above and through the process described in German Patent DE 12 55 660 in the name of Gabelein.

For example, the ozonized oil for uses according to the invention can be obtained by treating a starting fatty acid or oil with ozone gas up to reaching a concentration of ozone in the oil ranging from 12 to 60 mg, from 16 to 40 mg of ozone per gram of oil.

According to an aspect, the present invention relates to a composition comprising ozonized oil according to one or more of the embodiments mentioned hereinabove, and an appropriate carrier and/or excipient suitable for topical application.

The composition of the invention founds application in the pharmaceutical or cosmetic field, and is indicated for human or veterinary use.

According to some embodiments, the composition of the invention contains one or more additives.

For example, the composition of the invention contains one or more additives or substances selected from wetting agents, surfactants, emollients, soothing detergents, pH regulators, stabilizers, antimicrobials, emulsifiers, preservatives, thickeners, protective film forming agents, and optionally one or more active ingredients, such as antimicrobials, antihistamines, anti-inflammatory drugs. Each of these substances and/or additives may be present in a concentration, for example, from 0.001 to 10% by weight over the weight of the total composition. For example, the composition of the invention may contain 0.6% ozonized oil with ozone content of 0.5% and carriers, such as water, soy phospholipids, hydroxypropylmethylcellulose and polyanethylene biguanide.

By way of example, the composition of the invention may include as active ingredients one or more micro-elements or oligo-elements, such as zinc, magnesium, selenium, manganese, for example, in the form of salts, esters or hydroxides.

According to one embodiment, the composition may include vitamins and/or their derivatives and/or precursors, such as vitamins of the A, B, C, E, F group, e.g. at a concentration ranging from 0.001 to 25% by weight.

According to one preferred embodiment, the ozonized oil is carried by liposomes. According to this embodiment, liposomes are a suitable carrier for the ozonized oil, which increases disinfectant efficacy and compatibility with skin tissues in the periocular area and with the tissues of the ocular surface.

According to some embodiments, the composition of the invention is provided in liquid or semi-liquid form, suitable for local application, such as gel or solution to be applied to the skin area that needs treatment.

According to some embodiments, the composition of the invention comprises ozonized oil in an amount of 0.1 to 2%, 0.3 to 1%, 0.4 to 0.8% and one or more cosmetically or pharmaceutically acceptable carriers in an amount of 99.1 to 98% by weight.

According to another embodiment, the composition is provided in the form of a solution to be sprayed.

According to another embodiment, the composition of the invention is provided in solid or semi-solid form, for example, in the form of cream, ointment, paste, pomade, typically phospholipid emulsion.

The phospholipid composition or emulsion of the invention can be applied as a sanitizing, cleansing, emollient or soothing agent used to restore the physiological conditions of the cutaneous and/or subcutaneous structures of the ocular and periocular area exposed to external agents.

According to some embodiments, the ozonized oil or the phospholipid emulsion is applied directly to the tear film of the eye to reduce eye dryness.

According to some embodiments, the ozonized oil or the composition containing the ozonized oil of the invention is in the form of eyedrops, gel, emulsion, pomade or ointment to be applied to the skin of the periocular area to clean it. Within the scope of the invention, the term "phospholipid emulsion" or "emulsion of phospholipid" means an ozonized oil according to any one of the preceding embodiments which is carried by a phospholipid.

Alternatively, the ozonized oil or the composition containing the same can be applied on annexes of the eye to clean, sanitize or disinfect eyelids and eyelashes. In other embodiments, the ozonized oil or the composition that contains it can be instilled into the eye to obtain a cosmetic detergent or medical disinfectant effect.

In accordance with another aspect of the invention, a device is provided for the hygiene of the ocular and/or periocular area comprising a substrate and an ozonized composition according to any one of the embodiments described above.

According to some embodiments, the substrate is a fabric, for example, of cotton preferably hydrophilic cotton, a nonwoven fabric, such as polyester, or a polyurethane based substrate.

In some embodiments, the device is a handkerchief, a gauze, a wiping suitable for application to the ocular and/or periocular area.

In some embodiments, the device according to any one of the embodiments previously described is wetted or soaked with a composition according to any one of the preceding embodiments.

Typically, the device is a wet wipe with a composition containing an ozonized oil according to an embodiment of the invention and a cosmetically or pharmaceutically acceptable carrier.

According to some embodiments, the device of the invention is wetted with a quantity of ozonized oil of 0.02 to 0.06 g/cm², 0.03 to 0.05 g/cm².

Typically, the device wetted or soaked with ozonized oil is packed in an airtight container, such as an aluminum foil pouch or bag.

Preferably, the device is in sterile condition and/or under vacuum for medical or ophthalmic applications. Sterilization can be carried out according to conventional procedures in the pharmaceutical technique, for example, by exposure to ethylene oxide or gamma rays.

According to some embodiments, the device is a surgical medical device.

In the cosmetic field, the device of the invention can be applied for cleansing, or eyelid and/or eyelash lubrication. Once applied or rubbed on lashes and eyelids, the ozonized oil cleans the contact area by eliminating or reducing considerably the make-up remnants and atmospheric agents deposited during the day.

In the embodiments of the device in the form of gauze or handkerchief, it is appropriate to apply them on the eyelid surface while keeping the eye closed, possibly by circular movements that allow to clean the treated areas in depth and eliminate the impurities present on the epithelium while maintaining a natural moisturizing of the skin and underneath region.

In the medical field, ophthalmic use of the device in the ocular or periocular area sanitizes the treated area by reducing the present bacterial count. In addition, a germicidal action is combined with a soothing action that reduces redness due to the action of environmental agents or microorganisms developed therein. According to certain aspects, the device of the invention, particularly in the form of gauze wetted with ozonized oil, can be used in the medical field for the prevention and/or treatment of blepharitis, conjunctivitis, blepharo-conjunctivitis, or tear sac infection.

The term "native or starting oil" is used within the scope of the present invention with reference to the base oil, from which the ozonized oil originates, according to the invention.

The present invention will now be described with reference to the following examples, which are provided for illustrative purposes only and should not be understood as limiting the scope of the present invention.

The present invention will now be illustrated in detail with reference to the following examples.

### EXAMPLES

### Example 1

Process for the preparation of the ozonized oil identified in Table 1.

In a 20l generator, pure medical grade oxygen is introduced at the flow of 120 L/h; a 5000 volt electric discharge is applied to the generator, with formation of ozone reaching a concentration of about. 80-85 mg/L of O₃. The ozone is then bubbled into a reactor containing an oil having the fatty acid mixture of Example 2. The amount of ozone absorbed is controlled by conventional titration, and by measuring the density of the reaction mixture.

The linear correlation between the density of the reaction medium, expressed in g/L, and the peroxide index (IP) expressed in O₂/Kg equivalents was monitored.

### Example 2

Composition of a mixture of starting oils (fatty acids) from which the ozonized oil is obtained according to an embodiment of the invention.

| fatty acids | average % |
|---|---|
| *Monounsaturated* | **45.4%** |
| of which: | |
| Palmitoleic Acid C16:1 | 0.48% (per 100g of native product: 0.2g) |
| Oleic acid C18:1 | 99.52% (per 100g of native product: 45.3g) |
| *Polyunsaturated* | **40.1%** |
| of which: | |
| C16:2 | 2.54% (per 100g of native product: 0.3) |
| Linoleic acid C18:2 | 97.45% (per 100g of native product: 3938g) |
| Gamma linolenic acid C18:3 | About 0.4% (per 100g of native product: 0.15%) |
| *Saturated* | **10.1%** |
| of which: | |
| Palmitic Acid C16:0 | 53.4% (per 100g of native product: 5.4) |
| Stearic acid C18:0 | 34.6% (per 100g of native product: 3.5g) |
| Other C20 and C22 both unsaturated and saturated | Less than: 1% |

### Example 3

### Tests and experimental data

Eyedrops based on ozonized oil formulated with liposomes with the following formulation has been tested qualitatively and in vitro as to the ability to inhibit the growth of bacterial strains implicated in the most common ophthalmic diseases.

| **Fatty acids** | **average %** |
|---|---|
| *Monounsaturated* | **45.4%** |
| of which: | |
| Palmitoleic Acid C16:1 | 0.48% (per 100g of native product: 0.2g) |
| Oleic acid C18:1 | 99.52% (per 100g of native product: 45.3g) |
| *Polyunsaturated* | **40.1%** |
| of which: | |
| C16:2 | 2.54% (per 100g of native product: 0.3) |
| Linoleic acid C18:2 | 97.45% (per 100g of native product: 39.8g) |
| Gamma linolenic acid C18:3 | About 0.4% (per % of native product: 0.15%) |
| *Saturated* | **10.1%** |
| of which: | |
| Palmitic Acid C16:0 | 53.4% (per 100g of native product: 5.4) |
| Stearic acid C18:0 | 34.6% (per 100g of native product: 3.5g) |
| Other C20 and C22 both unsaturated and saturated | Less than: 1% |

In the composition of ozonized oil, fatty acids with chains C16 through C18 are most represented while fatty acids with chains C20 and C22 were present in a smaller quantity.

The ozonized oil used was contained in phospholipid micelles. It has been observed that liposome dispersion increases efficacy, as they combine with the cell membranes of the microorganisms and thus allow better contact of the ozonides.

The ozonisation reaction was carried out following the following scheme:

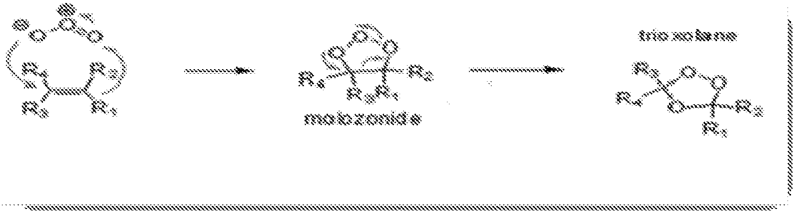

### Materials and Methods

The dissemination disks assay is a technique of spreading on inseminated agar for detection of the antimicrobial activity of chemotherapeutic agents.

The surface of agar plates, 6 mm filter paper disks, inseminated with a standard concentration of microorganisms (0.5 Mcfarland), were imbibed with 5 drops of eyedrops having the formulation reported above.

After 24 hours of incubation at 37°C, the presence of inhibition disks (halo around the paper disks) was evaluated.

The medium used was suitable for the type of test being carried out.

The following strains have been tested:
- *Staphyloccus aureus* , non-sporigenus, Gram-positive bacterium
- *Pseudomonas aeruginosa,* gram-negative bacterium;
- *Streptococcus pneumoniae,* gram-positive bacterium;

Eyedrop sample formulated with autoprotective phospholipids as previously illustrated
Incubated: 24 hrs at 37°C
Concentration of bacterial standards: 0.5 Mcfarland

### Results

| Strain | Plate bacterial development | Sensitivity |
|---|---|---|
| *Staphyloccus aureus* | Plentiful | Sensitive |
| *Pseudomonas aeruginosa* | Plentiful | Resistant |
| *Streptococcus pneumoniae* | Plentiful | Sensitive |

Bacterial strains of *Staphyloccus aureus* and *Streptococcus pneumoniae* were found to be sensitive to the eyedrops being tested, whereas the strains of *pseudomonas aeruginosa* were found resistant to the same.

### EXAMPLE 4

### Comparative test on the ozonized effect of ozonized oil and formulation of ozonized oil with liposomes of Example 3

### Purpose of the test

The "Crocin Bleaching Assay" (CBA) exhibits good stability and adaptability for the purpose of this comparative study. This method was proposed by Bors et al. (1984), subsequently modified by Tubaro et al. (1996) and applied on different matrices (Tubaro et al., 1998; Di Majo et al., 2005; Di Majo et al., 2008). Currently, it is the only essay present in literature that allows both the antioxidant and prooxidant action of a sample to be evaluated at the same time and it is applicable both on hydrophilic and lipophilic matrices.

The CBA method allows an evaluation of the antioxidant capacity of a substance or mixture by studying the crocin bleaching inhibitory kinetics (Bleaching Test). This method is based on the oxidation of crocin by peroxyl radicals (Figure 3, reaction [3]) produced by a radicalizing agent (Figure 3, reaction [1] and [2]). The oxidation of crocin occurs with a discoloration of the solution.

In the CBA method, the crocin bleaching speed is monitored over time by spectrophotometric readings at a specific wavelength of 443 nm and at a temperature of 40° C. The study is carried out two minutes after the addition of the ozonized agent (ozonized oil or solution containing ozonized oil in liposomal form) and for a 180 minute time span.

In the absence of antioxidant agent, peroxyl radicals react with the crocin poly-unsaturated hydrocarbon chain, crocetine esters (carotenoid extracted from saffron) by modifying its structural conjugation and inducing "bleaching" (whitening).

### Preparation of test formulations

An ozonized oil solution and a formulation (solution) containing ozonized oil in liposomal form are prepared.

### Spectrophotometric analysis of crocin bleaching kinetics

The spectrophotometric analysis of the crocin bleaching kinetics involves the preparation of 2 different reaction systems in 3 cuvets each: one system containing only native ozonized oil, the other only Ozodrop

**Table 1: Reaction systems made for the analysis of oxidising capacity.**

| **SYSTEMS** | **1A** | **2A** | **3A** | **4A** |
|---|---|---|---|---|
| µl CROCIN | 150 | 150 | 150 | 150 |
| µl H₂O | 850 | 700 | 770 | 830 |
| µl O₃ Oil | / | 150 | 80 | 20 |
| | | | | |

| **SYSTEMS** | **1B** | **2B** | **3B** | **4B** |
|---|---|---|---|---|
| µl CROCIN | 150 | 150 | 150 | 150 |
| µl H₂O | 850 | 700 | 770 | 830 |
| µl OZODROP | / | 150 | 80 | 20 |

The so filled cuvettes are incubated at 40° C in a thermoblock to maintain the optimum reaction conditions. Spectrophotometer readings at 443 nm are performed at 5 minutes, 10 minutes, 30 minutes, 60 minutes, and 180 minutes.

| OZONIZED OIL | ABS T0 | ABS T5 | ABS T10 | ABS T30 | ABS T60 | ABS T180 | % (FINAL ABS / INITIAL ABS) |
|---|---|---|---|---|---|---|---|
| 0 | 0.045 | 0.044 | 0.045 | 0.045 | 0.044 | 0.044 | 98% |
| 20 | 0.467 | 0.461 | 0.447 | 0.425 | 0,409 | 0.386 | 86% |
| 80 | 0.567 | 0.545 | 0.527 | 0.517 | 0.485 | 0.449 | 79% |
| 150 | 0.601 | 0.567 | 0.547 | 0.523 | 0.489 | 0.452 | 75% |

| OZONIZED OIL IN LIPOSOMAL FORM | ABS T0 | ABS T5 | ABS T10 | ABS T30 | ABS T60 | ABS T180 | % (FINAL ABS / INITIAL ASS) |
|---|---|---|---|---|---|---|---|
| 0 | 0.044 | 0.044 | 0.044 | 0.042 | 0.042 | 0.043 | 98% |
| 20 | 0.465 | 0.458 | 0.445 | 0,432 | 0.41 | 0.389 | 84% |
| 80 | 0.586 | 0.567 | 0.542 | 0.532 | 0.512 | 0.495 | 84% |
| 150 | 0,601 | 0.579 | 0.562 | 0.549 | 0.535 | 0.512 | 85% |

### Conclusions

The results of this test indicate that the formulation of ozonized oil in liposomal form has a less ozonized effect than ozonized oil. The less oxidative action on the tissues results in greater local tolerability of the liposomal ozonized oil formulation.

### EXAMPLE 5

### Comparative stability test

### 1. Purpose of the test

To compare the stability of a pure ozonized olein and a formulation containing ozonized oil in liposomal form.

The test involves determining the number of peroxides at time 0, time 90 and 180 days.

From the residual peroxide concentrations, the decay of the product is assessed. The product showing the lowest decay has a greater stability.

The number of peroxides is the quantity of substances present in the sample, expressed in milliequivalents of oxygen per kg, which oxidise potassium iodide under the conditions described.

This method consists of evaluating the content of the primary products of the substance self-oxidation by means of a iodometric titration. Titration of free iodine with a standardized sodium thiosulfate solution.

Reaction involved in titration:

S₂O₃ = + I₂ → S₄O₆ = + 21

| **Number of peroxides (temperature 25 °C)** | | | | |
|---|---|---|---|---|
| | | | | |

| *STEP* | **0 days** | **90 days** | **180 days** | **% Decay in 180 days** |
|---|---|---|---|---|
| ***Ozonized Oil*/** | **220** | **165** | **112** | **49%** |
| ***Ozonized Oil in liposomal form*** | **1.91** | **1.85** | **1.79** | **6%** |

### Conclusions

The results of this test indicate that the solution of ozonized oil in liposomal form has a greater stability than ozonized oil within 180 days.

### EXAMPLE 6

### OCULAR IRRITATION COMPARATIVE TEST ON OZONE IN LIPOSOMAL FORMULATION ON DOGS

### PURPOSE OF THE STUDY

The purpose of this study was to clinically evaluate the potential eye irritation of **a liposomal formulation containing ozonized oil according to example 3** in dogs spontaneously affected by conjunctivitis.

The study also provides a comparative eye irritation test to compare the liposomal formulation containing ozonized oil with that of non formulated ozonized oil

### MATERIALS AND METHODS

Three property dogs (2 males, 1 female, aged 6 years) spontaneously afflicted with bilateral conjunctivitis were enrolled in the study. Prior to enrollment, each dog has undergone a general clinical evaluation and a full ophthalmic evaluation, including the Schirmer Tear test, fluorescein staining, slit lamp examination, ophthalmoscopy and direct and indirect tonometry, to exclude any other cause of conjunctival inflammation.

Patients of the study were hospitalized at the Educational Veterinary Hospital of Camerino University; the dogs were housed in individual cages, cleaned before the animal's accommodation and then periodically. The room temperature and humidity were controlled by air conditioning units and kept constantly monitored. The general condition of the animals was assessed daily for the entire duration of the study (three days, beginning date 10 July 2017, ending date July 12, 2017).

Dogs were fed with standard feed and could drink tap water, filtered, *at libitum.* By the researcher's choice, the right eye received the ozonized oil, while the left eye received liposomal formulation (dispersion) containing ozone.

0.1 ml drop for each eye were placed in the conjunctival sac of each dog after gently pulling the lower flap away from the eyeball; after application, the eyelid has been released, giving the dog the chance to blink his eyelids to better distribute the treatment.

The eyes of each dog were examined shortly before application of the treatment and 5, 10, 30 minutes and 1, 24, 48 and 72 hours after instillation.

Table 1 contains parameters evaluated at each time point.

**Table 1. Parameters evaluated and marked for the detection of eye irritation.**

| **Conjunctivae Parameters redness** | **evaluation** |
|---|---|
| Normal vessels | 0 |
| Vases definitely injected above normal Single vessels that are not easily recognizable, of a | 1 |
| darker crimson red | 2 |
| Widespread bright red | 3 |

| **Chemosis** | |
|---|---|
| No swelling | 0 |
| Any swelling above normal | 1 |
| Obvious swelling with partial eyelid alteration | 2 |
| Swelling with half-closed eyelids | 3 |
| Swelling with half-closed to completely closed eyelids | 4 |

| **secretion** | |
|---|---|
| No secretion | 0 |
| Any quantity other than normal Secretion with umidificazione of eyelids and eyelashes | 1 |
| just around the eyelids Secretion with umidificazione of eyelids and eyelashes | 2 |
| and a considerable area around the eye | 3 |

The dogs included in the study had an evaluation 1 for one or more parameters at the time of enrollment; if the eye reached 2 or more for each evaluated reaction, these were considered as irritations.

### RESULTS

No change in score was observed after 5 minutes from eyedrop instillation neither for the ozonized oil nor for the liposomal dispersion containing ozone.

In 2/3 eyes that have received ozonized oil, irritation reactions have been reported. Chemosis was classified as degree 2 after 10 minutes in both eyes while only one eye was ranked degree 3 after 30 minutes, associated with a degree 3 chemosis.

In an eye that received the ozonized oil, 1 hour after treatment, the secretion was evaluated 2 and after 24 hours it was evaluated 3, in association with an increase in score for redness and chemosis, 3 and 4, respectively. In this case, the dog was withdrawn from the study due to an adverse reaction to the treatment.

No adverse reaction was recorded for the liposomal formulation (dispersion) containing ozone. In 2/3 of eyes, redness and chemosis have disappeared (from degree 1 to degree 0) 24 hours after treatment and have not been recorded any more while the remaining redness and eye chemosis have disappeared 48 hours after treatment.

### CONCLUSION

Based on the results, ozonized oil exhibits greater eye irritability than liposomal formulation (dispersion) containing ozone, which, on the contrary, significantly improved the initial clinical condition.

## Claims

1. Cosmetic non-therapeutic use of a composition containing ozonized oil for cleansing and/or lubrication of the ocular and/or periocular area, wherein said ozonized oil is carried by liposomes or phospholipid micelles and comprises ozonized fatty acids and/or fatty acid triglycerides selected from the group consisting of mono-unsaturated fatty acids C₁₄-C₂₄ polyunsaturated fatty acids C₁₈-C₂₂ and mixtures thereof and/or their triglycerides with from 1 to 3 glycerol molecules.

2. Use in accordance with claim 1, wherein said ozonized monounsaturated fatty acids are C₁₆ - C₂₂ or C₁₆ - C₁₈.

3. Use in accordance with claim 1 or 2, wherein ozone of ozonized oil has a concentration in the range of 12 to 60 mg, preferably of 16 to 40 mg per gram of oil.

4. Use according to any one of claims 1-3, wherein said liposomes or phospholipid micelles comprise phospholipids preferably containing phosphatidylcholine.

5. Use according to any one of claims 1-4, wherein liposomes comprise phospholipid vesicles having a diameter of 5 to 2000 nm.

6. Use according to any one of claims 1-5, wherein liposomes contain ozonides in a concentration of 3% to 60% by weight (w/w), preferably of 6 to 30% by weight, with respect to the other components of the phospholipid.

7. A composition comprising ozonized oil for use in the disinfection and/or healing of an epithelial or keratin structure of the eye or the periocular area, wherein said ozonized oil is carried by liposomes or phospholipid micelles and comprises ozonized fatty acids and/or fatty acid triglycerides selected from the group consisting of mono-unsaturated fatty acids C₁₄-C₂₄ polyunsaturated fatty acids C₁₈-C₂₂ and mixtures thereof and/or their triglycerides with from 1 to 3 glycerol molecules.

8. A composition for use in accordance with claim 7, wherein said ozonized monounsaturated fatty acids are C₁₆ - C₂₂ or C₁₆ - C₁₈.

9. A composition for use in accordance with claim 7 or 8, wherein ozone of ozonized oil has a concentration in the range of 12 to 60 mg, preferably of 16 to 40 mg per gram of oil.

10. A composition for use according to any one of claims 7-9, wherein said liposomes or phospholipid micelles comprise phospholipids containing phosphatidylcholine.

11. A composition for use according to any one of claims 7-10, wherein liposomes comprise phospholipid vesicles having a diameter of 5 to 2000 nm.

12. A composition for use according to any one of claims 7-11, wherein liposomes contain ozonides in a concentration of 3% to 60% by weight (w/w), preferably of 6 to 30% by weight, with respect to the other components of the phospholipid.

13. A device comprising a solid support wetted with an ozonized oil wherein said ozonized oil is carried by liposomes or phospholipid micelles and comprises ozonized fatty acids and/or fatty acid triglycerides selected from the group consisting of mono-unsaturated fatty acids C₁₄-C₂₄ polyunsaturated fatty acids C₁₈-C₂₂ and mixtures thereof and/or their triglycerides with from 1 to 3 glycerol molecules.

14. A device in accordance with claim 13, **characterised in that** said solid support is made from a material selected from cotton wool, non-woven fabric, polyurethane and is preferably wetted with an amount of ozonized oil of 0.02 to 0.06 g/cm².

## Patentansprüche

1. Kosmetische nichttherapeutische Verwendung einer Zusammensetzung, die ozonisiertes Öl enthält, zur Reinigung und/oder Einfettung des okularen und/oder periokularen Bereichs, wobei das ozonisierte Öl von Liposomen oder Phospholipidmizellen mitgetragen wird und ozonisierte Fettsäuren und/oder Fettsäuretriglyceride umfasst, die aus der Gruppe ausgewählt sind, die aus einfach ungesättigten C₁₄-C₂₄-Fettsäuren, mehrfach ungesättigten C₁₈-C₂₂-Fettsäuren und Gemischen davon und/oder deren Triglyceriden mit 1 bis 3 Glycerinmolekülen besteht.

2. Verwendung gemäß Anspruch 1, wobei die ozonisierten einfach ungesättigten Fettsäuren C₁₆ - C₂₂ oder C₁₆ - C₁₈ sind.

3. Verwendung gemäß Anspruch 1 oder 2, wobei das Ozon vom ozonisierten Öl eine Konzentration im Bereich von 12 bis 60 mg, vorzugsweise von 16 bis 40 mg pro Gramm Öl, aufweist.

4. Verwendung gemäß einem der Ansprüche 1-3, wobei die Liposomen oder Phospholipidmizellen Phospholipide umfassen, die vorzugsweise Phosphatidylcholin enthalten.

5. Verwendung gemäß einem der Ansprüche 1-4, wobei die Liposomen Phospholipidvesikel umfassen, die einen Durchmesser von 5 bis 2000 nm aufweisen.

6. Verwendung gemäß einem der Ansprüche 1-5, wobei die Liposomen Ozonide in einer Konzentration von 3 bis 60 Gew.-% (w/w), vorzugsweise von 6 bis 30 Gew.-%, bezogen auf die anderen Bestandteile des Phospholipids, enthalten.

7. Zusammensetzung, umfassend ozonisiertes Öl zur Verwendung bei der Desinfektion und/oder Heilung einer Epithel- oder Keratinstruktur des Auges oder des periokularen Bereichs, wobei das ozonisierte Öl von Liposomen oder Phospholipidmizellen mitgetragen wird und ozonisierte Fettsäuren und/oder Fettsäuretriglyceride umfasst, die aus der Gruppe ausgewählt sind, die aus einfach ungesättigten C₁₄-C₂₄-Fettsäuren, mehrfach ungesättigten C₁₈-C₂₂-Fettsäuren und Gemischen davon und/oder deren Triglyceriden mit 1 bis 3 Glycerinmolekülen besteht.

8. Zusammensetzung zur Verwendung gemäß Anspruch 7, wobei die ozonisierten einfach ungesättigten Fettsäuren C₁₆ - C₂₂ oder C₁₆ - C₁₈ sind.

9. Zusammensetzung zur Verwendung gemäß Anspruch 7 oder 8, wobei das Ozon vom ozonisierten Öl eine Konzentration im Bereich von 12 bis 60 mg, vorzugsweise von 16 bis 40 mg pro Gramm Öl, aufweist.

10. Zusammensetzung zur Verwendung gemäß einem der Ansprüche 7-9, wobei die Liposomen oder Phospholipidmizellen Phospholipide umfassen, die Phosphatidylcholin enthalten.

11. Zusammensetzung zur Verwendung gemäß einem der Ansprüche 7-10, wobei die Liposomen Phospholipidvesikel umfassen, die einen Durchmesser von 5 bis 2000 nm aufweisen.

12. Zusammensetzung zur Verwendung gemäß einem der Ansprüche 7-11, wobei die Liposomen Ozonide in einer Konzentration von 3 bis 60 Gew.-% (w/w), vorzugsweise von 6 bis 30 Gew.-%, bezogen auf die anderen Bestandteile des Phospholipids, enthalten.

13. Vorrichtung, umfassend einen festen Träger, der mit einem ozonisierten Öl benetzt ist, wobei das ozonisierte Öl von Liposomen oder Phospholipidmizellen mitgetragen wird und ozonisierte Fettsäuren und/oder Fettsäuretriglyceride umfasst, die aus der Gruppe ausgewählt sind, die aus einfach ungesättigten C₁₄-C₂₄-Fettsäuren, mehrfach ungesättigten C₁₈-C₂₂-Fettsäuren und Gemischen davon und/oder deren Triglyceriden mit 1 bis 3 Glycerinmolekülen besteht.

14. Vorrichtung gemäß Anspruch 13, **dadurch gekennzeichnet, dass** der feste Träger aus einem Material hergestellt ist, das aus Watte, Vliesstoff, Polyurethan ausgewählt ist, und vorzugsweise mit einer Menge an ozonisiertem Öl im Bereich von 0,02 bis 0,06 g/cm² benetzt ist.

## Revendications

1. Utilisation cosmétique non-thérapeutique d'une composition contenant de l'huile ozonisée pour nettoyer et/ou lubrifier l'œil et/ou la zone périoculaire, où ladite huile ozonisée est portée par des liposomes ou des micelles de phospholipides et comprend des acides gras ozonisés et/ou des triglycérides d'acides gras choisis parmi le groupe composé d'acides gras monoinsaturés en C₁₄-C₂₄ d'acides gras polyinsaturés en C₁₈-C₂₂ et des mélanges de ceux-ci et/ou leurs triglycérides avec de 1 à 3 de molécules de glycérol.

2. Utilisation selon la revendication 1, où lesdits acides gras monoinsaturés ozonisés sont en C₁₆ - C₂₂ ou C₁₆ - C₁₈.

3. Utilisation selon la revendication 1 ou 2, où l'ozone d'huile ozonisée a une concentration comprise entre 12 et 60 mg, de préférence de 16 à 40 mg par gramme d'huile.

4. Utilisation selon l'une quelconque des revendications 1 à 3, où lesdits liposomes ou micelles de phospholipides comprennent des phospholipides contenant de préférence de la phosphatidylcholine.

5. Utilisation selon l'une quelconque des revendications 1 à 4, où des liposomes comprennent des vésicules de phospholipides ayant un diamètre de 5 à 2000 nm.

6. Utilisation selon l'une quelconque des revendications 1 à 5, où des liposomes contiennent des ozonides en une concentration de 3% à 60% en poids (p/p), de préférence de 6 à 30% en poids, par rapport aux autres composants du phospholipide.

7. Composition comprenant de l'huile ozonisée pour une utilisation dans la désinfection et/ou guérison d'un épithélial ou d'une structure kératinique de l'œil ou de la zone périoculaire, où ladite huile ozonisée est portée par des liposomes ou des micelles de phospholipides et comprend des acides gras ozonisés et/ou des triglycérides d'acides gras choisis parmi le groupe composé d'acides gras monoinsaturés en C₁₄-C₂₄ d'acides gras polyinsaturés en C₁₈-C₂₂ et des mélanges de ceux-ci et/ou leurs triglycérides avec de 1 à 3 de molécules de glycérol.

8. Composition pour une utilisation selon la revendication 7, où lesdits acides gras monoinsaturés ozonisés sont en C₁₆ - C₂₂ ou C₁₆ - C₁₈.

9. Composition pour une utilisation selon la revendication 7 ou 8, où l'ozone de l'huile ozonisée a une concentration comprise entre 12 et 60 mg, de préférence de 16 à 40 mg par gramme d'huile.

10. Composition pour une utilisation selon l'une quelconque des revendications 7 à 9, où lesdits liposomes ou micelles de phospholipides comprennent des phospholipides contenant de la phosphatidylcholine.

11. Composition pour une utilisation selon l'une quelconque des revendications 7 à 10, où des liposomes comprennent des vésicules de phospholipides ayant un diamètre de 5 à 2000 nm.

12. Composition pour une utilisation selon l'une quelconque des revendications 7 à 11, où des liposomes contiennent des ozonides en une concentration de 3% à 60% en poids (p/p), de préférence de 6 à 30% en poids, par rapport aux autres composants du phospholipide.

13. Dispositif comprenant un support solide mouillé avec une huile ozonisée où ladite huile ozonisée est portée par des liposomes ou des micelles de phospholipides et comprend des acides gras ozonisés et/ou des triglycérides d'acides gras choisis parmi le groupe composé d'acides gras monoinsaturés en C₁₄-C₂₄ d'acides gras polyinsaturés en C₁₈-C₂₂ et des mélanges de ceux-ci et/ou leurs triglycérides avec de 1 à 3 de molécules de glycérol.

14. Dispositif selon la revendication 13, **caractérisé en ce que** ledit support solide est constitué d'un matériau choisi parmi du coton, du tissu non tissé, du polyuréthane et est de préférence mouillé avec une quantité d'huile ozonisée de 0,02 à 0,06 g/cm².
